Europäisches Patentamt

European Patent Office

Office européen des brevets

(11) Veröffentlichungsnummer: **0 008 620**
**B1**

(12) **EUROPÄISCHE PATENTSCHRIFT**

(45) Veröffentlichungstag der Patentschrift:
30.12.81

(21) Anmeldenummer: **79102091.0**

(22) Anmeldetag: **25.06.79**

(51) Int. Cl.³: **A 61 F 1/00**

(54) Sehnen- und Bänderersatz unter Verwendung von Kohlenstoffäden.

(30) Priorität: **24.08.78 DE 2836921**

(43) Veröffentlichungstag der Anmeldung:
**19.03.80 Patentblatt 80/6**

(45) Bekanntmachung des Hinweises auf die Patenterteilung:
**30.12.81 Patentblatt 81/52**

(84) Benannte Vertragsstaaten:
**CH FR GB SE**

(56) Entgegenhaltungen:
**DE-A1-2 827 006**
**US-A-3 513 484**
**US-A-3 526 005**
**US-A-3 526 906**
**US-A-3 992 725**

(73) Patentinhaber: **SIGRI ELEKTROGRAPHIT GMBH, Werner von Siemens-Strasse 18, D-8901 Meitingen (DE)**

(72) Erfinder: **Claes, Lutz, Dr., Kolpingstrasse 12, D-7914 Pfuhl bei Ulm (DE)**
Erfinder: **Wolter, Dietmar, Prof. Dr., Ihlendieksweg 30, D-2070 Grosshansdorf (DE)**

Sehnen- und Bänderersatz unter Verwendung von Kohlenstoffäden

Die Erfindung betrifft einen Sehnen- und Bänderersatz unter Verwendung von Kohlenstoffäden.

Verletzte Sehnen und Bänder heilen bei einer primären konservativen Behandlung oder nach einer späten Operation häufig nur ungenügend, so dass die ursprüngliche Funktion oftmals nicht mehr erreicht wird und beispielsweise im Bereich des Kniegelenks Instabilitäten entstehen, die den natürlichen Verschleiss des Gelenks wesentlich verstärken. Der Endzustand dieses Prozesses ist nicht selten eine Arthrose. Zur Vermeidung derartiger schwerer Schäden ist vorgeschlagen worden, die nichtfunktionsfähigen Bänder und Sehnen durch körpereigenes Gewebe zu ersetzen. Bei den Transplantaten nimmt jedoch während der Umbauphase unabhängig von der primären Stabilität die Festigkeit des Gewebes ab, so dass eine sehr lange postoperative Ruhigstellung nötig ist, die zu bleibenden Versteifungen führen kann.

Alloplastische Ersatzstoffe weisen diesen Nachteil nicht auf und es sind daher zahlreiche Stoffe, wie Stahldrähte und Polyamid-, Polyester- und Seidenfäden, als Ersatz für zerrissene Bänder und Sehnen vorgeschlagen worden. Die teilweise unzureichende Gewebeverträglichkeit, das verschiedene elastische Verhalten und die Oberflächenbeschaffenheit dieser Stoffe verhinderten jedoch eine befriedigende Lösung des Organersatzes. Die Fäden liegen im allgemeinen als Faserbündel vor. Da die Faserbündel sich nur schwierig befestigen lassen, sind auch bänderförmige Gebilde vorgeschlagen worden, deren Ende in Metallösen geklammert sind (US-A-3513484). Günstiger als synthetische Fasern verhalten sich Kohlenstoffäden, z.B. sind durch US-A- 3526906 aus Kohlenstoffasern bestehende Blutgefässe bekannt, die naturgemäss vergleichsweise geringen dynamischen Belastungen ausgesetzt sind. Es ist schliesslich bekanntgeworden, Kohlenstoffäden in der Form von Tauen als Ersatz von Bändern und Sehnen zu verwenden. Unter dem Begriff «Kohlenstoffäden», die durch Pyrolyse von faserförmigen Naturstoffen oder synthetischen Faserstoffen erzeugt werden, sollen im folgenden ebenfalls Graphitfäden verstanden werden. Kohlenstoffäden sind gewebeverträglich, histologische Untersuchungen zeigten kaum Entzündungsreaktionen nach mehrmonatiger Implantationszeit, die Festigkeit ist ausserordentlich hoch und sie induzieren die Bildung von gerichtetem kollagenen sehnenartigen Bindegewebe. Innerhalb einiger Monate ist dieser Regenerationsprozess abgeschlossen und ein faserreiches Gewebe gebildet worden.

Kohlenstoffäden weisen jedoch einen grösseren Elastizitätsmodul auf als Sehnen und Bänder und durch das wesentlich verschiedene elastische Verhalten wird vor allem die Beweglichkeit von Gelenken beeinträchtigt, deren Bänder durch Kohlenstoffasern ersetzt sind. Es ist daher Aufgabe der Erfindung, aus Kohlenstoffasern einen alloplastischen Sehnen- und Bänderersatz zu schaffen, der im wesentlichen das gleiche elastische Verhalten wie körpereigenes Gewebe aufweist.

Die Aufgabe wird erfindungsgemäss dadurch gelöst, dass die Kohlenstoffäden ein bandförmiges Gewebe bilden, bei welchem die Schussfäden in Richtung der längeren Kante verlaufen und eine kleinere Filamentzahl als die Kettfäden aufweisen. Zweckmässig beträgt die Filamentzahl der Schussfäden etwa 2000 bis 10000 und der Kettfäden > 12000. Für die ossäre Verankerung des Gewebebandes ist es von Vorteil, die kurzen Gewebekanten saumartig zu verstärken, beispielsweise durch Umnähen der Bandkante oder eine entsprechende Verkürzung der Bandbreite. Zur Verbesserung der Scherfestigkeit der Kohlenstoffäden ist es schliesslich vorteilhaft, in an sich bekannter Weise die Filamente mit einer dünnen Pyrokohlenstoffschicht zu beschichten.

Gewebebänder aus Kohlenstoffäden weisen als alloplastischer Ersatz von Sehnen und Bändern zusätzlich zu den oben beschriebenen Vorteilen von Kohlenstoffäden für diesen Zweck etwa die gleiche Elastizität wie das zu ersetzende körpereigene Gewebe auf, so dass die Beweglichkeit von Gelenken, die derartige Ersatzbänder enthalten, nicht beeinträchtigt wird. Der Abbau der Kohlenstoffäden des Implantats und deren Ersatz durch neugebildetes körpereigenes Gewebe verläuft schliesslich im wesentlichen ohne Veränderung des elastischen Verhaltens und entsprechend ohne Beeinträchtigung der Gelenkfunktion.

Die Erfindung wird im folgenden anhand einer Zeichnung beispielhaft erläutert.

Dargestellt ist die Verwendung eines bandförmigen Gewebes aus Kohlenstoffäden als Ersatz des medialen Knieseitenbands 1. Die Schussfäden, deren Filamentzahl 3000 beträgt, verlaufen in Richtung der langen Gewebekanten; die quer dazu verlaufenden Kettfäden weisen 20000 Filamente auf. Die stärkeren Kettfäden bewirken insbesondere eine bessere Rückstellung des mechanisch entlasteten Gewebebandes, dessen kürzere Gewebekanten 2 saumartig verstärkt sind. Die Enden des Gewebebands 1 sind in kanalartigen Ausnehmungen 3 und 3' geführt und durch Schrauben 4 und 4' im Tibia 5 und Femur 6 verankert, zwischen denen sich der Kniegelenkspalt 7 erstreckt.

Histologische Untersuchungen nach einer Implantationszeit von 6 Wochen zeigten eine vollständige Ummantelung der einzelnen, einen Durchmesser von 6 bis 8 µm aufweisenden Filamente mit einem zellreichen Körpergewebe. Nach etwa 3 Monaten hat sich ein in Richtung der Filamentlängserstreckung verlaufendes Sehnengewebe gebildet und die Kohlenstoffilamente sind zu einem Teil zerbrochen. Die Filamente sollten naturgemäss erst nach der Neubildung des Sehnengewebes zerfallen oder abgebaut werden. Die Standzeit kann, falls erforderlich, durch eine Beschichtung der Filamente mit Pyrokohlenstoff verlängert werden.

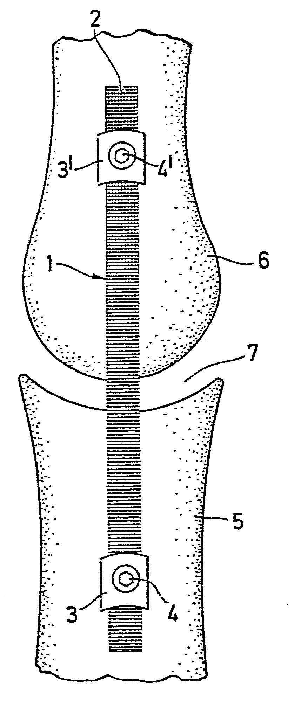

## Patentanspruch

Sehnen und Bänderersatz unter Verwendung von Kohlenstoffäden, dadurch gekennzeichnet, dass die Kohlenstoffäden ein bandförmiges Gewebe bilden, bei welchem die Schussfäden in Richtung der längeren Kante verlaufen und eine kleinere Filamentzahl als die Kettfäden aufweisen.

## Claims

1. Artificial tendon or ligament comprising carbon fibers wherein said fibers are forming a tape-like fabric wherein the weft yarn is orientated parallel to the longer edges of the fabric and consists of less filaments than the warp yarns.

## Revendications

1. Substitut de tendons et ligaments avec utilisation de fibres de carbone, caractérisé en ce que les fibres de carbone forment un tissu en forme de bande (1), dans laquelle les fils de trame sont disposés dans le sens de la longueur et présentent un nombre de filaments inférieur à celui des fils de chaîne.